# EUROPEAN PATENT APPLICATION

(11) **EP 0 521 211 A1**
(43) Date of publication of application: **07.01.1993**
(21) Application number: 91306148.7
(22) Date of filing: 05.07.1991
(51) Int. Cl.: A61M 1/00, A61M 3/00, A61C 17/024

(54) **Pressurised fluid dispensing device**

(71) Applicant: SMITH & NEPHEW INC., Largo, Florida 34643 (US)
(72) Inventor: Scheuble, Gustav A., Des Plaines, Illinois 60016 (US); Malmin, Oscar, Boise, Idaho 83705 (US)
(74) Representative: Leckey, David Herbert

(57) **Abstract**

A pressurised fluid dispensing apparatus (10) comprising a reservoir (22) fillable through a closable circular port (24) and being adapted to receive a plunger means (28) and communicating with first check valve means (30) for providing compressed air to dispense fluid from said reservoir (22) through a filtered discharge channel (32), said channel (32) being connected to second check valve means (34) disposed in said fluid placement device to regulate flow of said fluid.

## Description

The invention of this application relates to a pressurised fluid dispensing apparatus. More particularly, this invention relates to a pressurised fluid dispensing apparatus for use with a precision-controlled fluid placement device.

Various fluid dispensing pumps and sprayers have been previously suggested. Keyes et al., US Patent No. 4700872 describes a pump for dispensing liquid via a syringe in which a hollow cylinder is mounted. The cylinder is connected to the chamber by a first normally closed check valve. A hollow plunger, which receives a liquid charge, reciprocates in the cylinder and is connected thereto by a second normally closed check valve. On the out-stroke of a piston, the liquid in the piston is sucked through the second normally closed check valve into the cylinder and, on the in-stroke of the plunger, the liquid is forced through the normally closed check valve into the chamber wherein it is maintained under pressure created by the pumping action of the plunger. An outlet tube leads from the lower part of the chamber to a syringe and a normally closed pinch valve in the syringe is manually operable to permit liquid under pressure to be squirted from the syringe.

Spengler et al, US Patent No. 4537334 describes a reinforced plastic bottle or vessel with a relatively large neck with buttressed threads and a bevelled rim on its neck onto which threads is screwed a cap assembly. The cap assembly comprises a plastic cap base member having an integral handle and a nozzle connection, a plastic manual air pump, a plastic siphon, a valve and spray nozzle assembly, and a lever to operate the valve. The air pump is supported by an integral flange and it depends vertically from the cap base member into the bottle with a disc-type check valve at its bottom.

Cohen, US Patent No. 4175704 describes a non-aerosol type spray device comprising a container for housing a liquid material to be dispensed and a plunger slideable as a piston within the container and an elongate tubular member for actuating the plunger with a bore extending continuously therethrough for communication with the interior of the container at one end. There is additional communication with a spray nozzle at the other end for passage of the liquid material from the container through the bore to the spray nozzle in response to displacement of the plunger.

Genese, US patent No. 4036232 describes an aspiration unit which affords a uniform suction level for aspirating fluids from body cavities. The unit comprises a double piston member, a first head of which is in sealing engagement with a syringe barril and the second head of which is oppositely disposed and contained in a telescoping member which is also receivable in the syringe barrel. Upon movement of the telescoping member outwardly from the syringe barrel, a vacuum will be created in the telescoping member as well as in that portion of the barrel member between the first piston member and the nozzle which is closed from the atmosphere by means of a valve. Upon insertion of a flexible tube into a body cavity and opening the valve, fluid will be aspirated into the syringe barrel by means of the partial vacuum created in the syringe barrel and in the telescoping member.

Rodrigues, Jr., US Patent No. 3191807 describes a liquid dispensing device for dispensing ultra-micro volumes of liquid. The dispensing device comprises a syringe having a longitudinal passageway for a piston assembly and includes an enlarged recess at it upper end. The passageway leads to an intake-discharge member. The flow into the chamber is controlled by an intake valve assembly and a discharge valve assembly controls the flow from the chamber. The piston assembly in the passageway has a piston adapted to alter the effective volume of the intake-discharge chamber during reciprocation. The stroke of the piston assembly is adjustably controlled by a plunger.

Esmay US Patent No. 2880939 describes a device for spraying liquid materials in a finally divided or atomised form. The spraying devices comprises an outer, elongated cylindrical tube made of transparent material and a plunger supply tube also made of a transparent material loosely telescoped within the upper end of the outer tube and having a length of the same order of magnitude as the outer tube. The plunger tube contains the material to be sprayed and the bottom of the plunger tube contains a spring-urged valve assembly which, when the plunger tube is fully extended into the outer tube, fully opens to dispense a fixed quantity of material into a receiving well at the bottom of the plunger tube.

Pollak, US Patent NO. 2743847, describes a mixing and dispensing apparatus for mixing and dispensing a liquid antiseptic with quantities of water. The apparatus consists of a cylindrical container which is adapted to be secured to a water outlet and which contains a main mixing and water storing chamber, a liquid chemical storage chamber mounted above the mixing chamber, means for dispensing and mixing a measured quantity of liquid chemical solution of water in the main chamber and control means controlling the entry of water and discharge of a mixture of water and liquid chemical solution, respectively.

Hein, US Patent No. 1563627, describes a hypodermic syringe construction wherein the solution to be ejected is adapted to be contained within a phial or ampule which is used as a piston within the syringe in filling the syringe and ejecting the fluid therefrom.

Heretofore, none of the fluid dispensing devices adequately simplified precise applications of liquids without requiring that pressurising means be removed from a reservoir in which the liquid material is maintained. The invention disclosed herein comprises a pressurised fluid dispensing apparatus which overcomes the significant deficiencies of prior fluid dispensing devices and is able to precisely and controllably supply solutions in dentistry, medicine, veterinary medicine, industrial and commercial fabrications and various other uses.

The present invention contemplates a pressurised fluid dispensing means using a precision fluid placement means for precise, controlled administration of fluids.

In one aspect of the present invention, the pressurised fluid dispensing means of the invention comprises at least one fluid reservoir chamber fillable through a port which is closeable by a removable cap. The fluid reservoir chamber is adapted to receive a plunger means in an integral chamber for providing compressed air through a first check valve means to the fluid reservoir chamber with the compressed air acting to dispense fluid from the fluid reservoir chamber through a filter discharge channel, which has a second check valve means. The discharge channel is further connected to a second check valve means. The discharge channel is further connected to a check valve means disposed in the precision fluid placement means to manually regulate fluid flow.

In a further aspect of the present invention, the pressurised fluid dispensing means of the invention includes attachment means for securing a precision-controlled fluid placement means in the form of a handpiece assembly thereto.

In an additional aspect of the present invention, the plunger means of the pressurised fluid dispensing means of the invention includes a manually operated plunger shaft which has a head and is slideable within the integral chamber of the fluid reservoir chamber. The plunger shaft is disposed in the chamber between a plurality of guide means for maintaining the shaft slidably within the plunger chamber.

In another aspect of the present invention, the fluid reservoir chamber may have a threaded port to receive a threaded plunger containing the manually operated plunger shaft which has a head and is slideable within the removable plunger chamber as the plunger means of pressurising the fluid reservoir chamber.

In a further aspect of the present invention, the pressurised fluid dispensing means of the invention includes a filtered dispensing tube communicating through the removable cap with an attachment means for securing a precision-controlled fluid placement means in the form of a handpiece assembly thereto.

In an additional aspect of the present invention, the precision-controlled fluid placement handpiece assembly means includes a handpiece having a tube connecting to the nose piece with an integral cannula.

In a further aspect of the present invention, the precision-controlled fluid placement handpiece assembly means includes a handpiece having distal connector means for connection with removable cannulas and needles, and a proximal connector means for connection to the attachment means of the pressurised fluid dispensing means.

The present invention provides several benefits and advantages.

One of the benefits of the present invention is that the pressurised fluid dispensing apparatus of the invention provides a reservoir means for the pressurised dispensing of various kinds of solutions, fluids or liquids.

Another benefit of the present invention is that the pressurised fluid dispensing apparatus of the invention provides a quick connection that allows the joining of tubing to the pressurised reservoir and to a manually controlled handpiece designed to precisely deliver desired amounts of solutions, liquids or fluids into specific sites or locations. For example, in living bodies, the pressurised fluid dispensing apparatus of the invention is adaptable to common dental cleansing usages such as the lavage of root canal preparations, periodontal pockets, gingival sulci, furcations, implant sockets, extraction sockets, infected sockets, pericoronal infections or other sites or infected areas in the oral cavity. Additionally, the apparatus of the invention may be used to perform various medical or veterinary-lavaging treatments for infections in the nostrilla, external auditory meatuses, draining fistula or other infected areas in mammalian bodies. The apparatus of the invention is further adaptable to many common commercial or industrial uses, such as the precise application of adhesives or lubricants.

A further benefit of the present invention is that the pressurised fluid dispensing apparatus of the invention permits simplified, precise applications of liquids, solutions or fluids and does not require that the pressurising pump means be removed from the reservoir.

One of the advantages of the present invention is that the pressurised fluid dispensing apparatus of the invention provides a complete mobile self-contained pressurised dispensing device that does not rely upon any form of electrical power or motors or connections with sources of electricity, fluids or compressed air and has a minimum number of movable parts.

Another advantage of the present invention is that the pressurised fluid dispensing device of theinvention may be economically manufactured through, for example, a moulding process employing elastomeric materials, such as plastics, or through machining and casting processes using metals, plastics or combinations thereof.

Yet another advantage of the present invention is that the pressurised fluid dispensing device of the invention permits safe, highly efficient, effective and diversified uses.

Other benefits and advantages of the present invention will become readily apparent to those skilled in the art from the following detailed description of the invention, the drawings and the appended claims.

In drawings forming a portion of the disclosure of this invention:
Figure 1 is a perspective view of the pressurised fluid dispensing device of the present invention;
Figure 2 is a vertical cross-sectional view of the reservoir and integral chamber thereof of the pressurised fluid dispensing device of the present invention;
Figure 3 is a horizontal cross-sectional view of the bottom portion of the pressurised fluid dispensing device of the present invention;
Figure 4 is a cross-sectional view of the integral chamber of the pressurised fluid dispensing apparatus of Figure 3 illustrating the plunger means of the present invention;
Figure 5 is a perspective view of the base of the integral chamber illustrated in Figure 3; and
Figure 6 is a perspective view of a portion of the base of the reservoir of Figure 3 illustrating the base of the pressurised fluid dispensing device of the present invention.

The present invention is directed to a pressurised fluid dispensing apparatus for use with a precision-controlled fluid placement device, such as a handpiece assembly. Applicants have previously filed Disclosure Document No. 230749 on July 10th, 1989, a copy of which is attached hereto as Exhibit 1 and incorporated herein by reference.

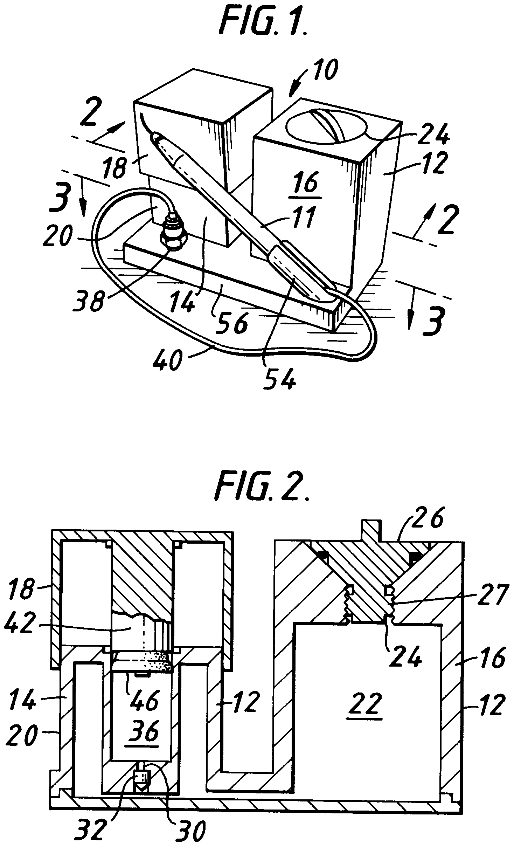

With reference to Figures 1-6, a preferred embodiment of the pressurised fluid dispensing apparatus of the present invention is shown. Pressurised fluid dispensing apparatus 10 for use with a precision-controlled fluid placement device, such as a dental handpiece 11, comprising a housing 12, made of suitable plastic or other elastomeric mouldable materials well known to those of skill in the art. Alternatively, housing 12 may be cast or machined of metals, plastics or combinations thereof. Housing 12 has a left generally rectangular portion 14 and a right generally rectangular portion 16. Left portion 14 has a top section 8 overlying a bottom section 20.

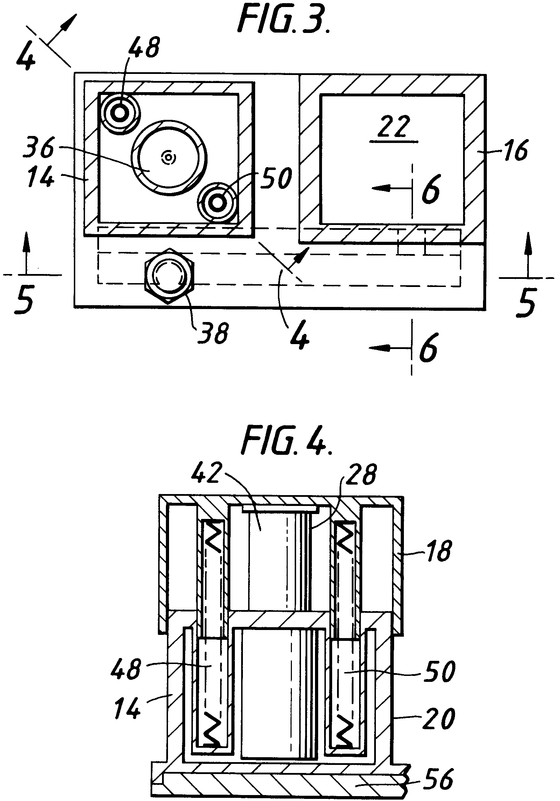

As shown in Figure 2, right portion 16 of housing 12 contains a fluid reservoir 22 which is fillable with any desired fluid or liquid through a circular port 24. Circular port 24 is closeable by a removable cap 26 and is threaded with threads 27 to receive removable cap 26 which, when screwed into port 24, hermetically seals reservoir 22 to prevent air entering into it. Reservoir 22 extends into left portion 14 of housing 12 and is adapted to receive plunger means 28 as shown in Figures 2 and 4, and to communicate with a first check valve means 30 for providing compressed air to dispense the fluid from the reservoir through a filtered discharge channel 32 as will be discussed in detail hereinafter. Channel 32 is connected to a second check valve means 34 disposed in fluid placement device 11 to regulate the flow of the fluid and to prevent leakage of such fluid when apparatus 10 is not in use.

Reservoir 22 further includes, in left portion 14 of housing 12, an integral chamber 36 to receive plunger means 28.

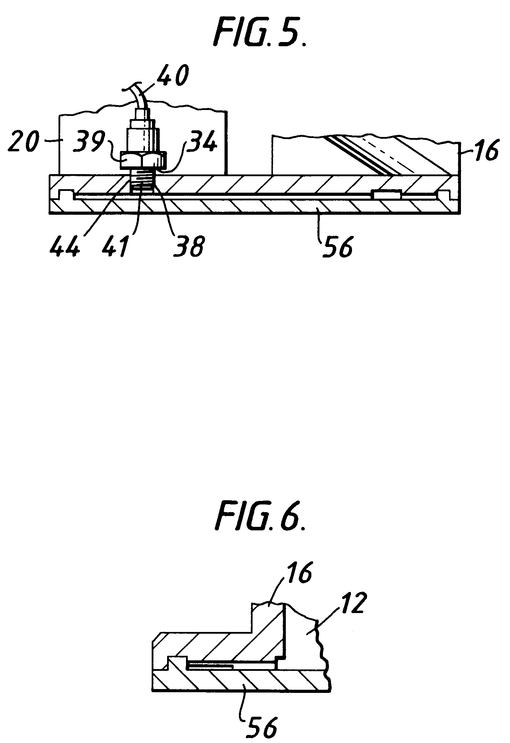

The pressurised fluid dispensing apparatus 10 of the present invention has an attachment means 38, such as a nozzle, as shown in Figures 3 and 5, for securing handpiece assembly 11 to the apparatus 10. Handpiece assembly 11 has a male member 39 which engages attachment means 38. Member 39 is connected to the handpiece assembly 11 by means of tubing 40 through which fluid flows. Attachments means 38 is threaded with threads on female valve post 44 to accommodate member 39 and secure it in the desired position. Attachment means 38 is preferably located at the lowest internal level of integral chamber 36.

The plunger means 28 in chamber 36 comprises a plunger shaft 42 having a head 46 of rubberised material. Shaft 42 is slideable within chamber 36 to exert pressure upon the fluid in reservoir 22 as will be discussed below. Shaft 42 is disposed in chamber 36 between generally cylindrical guide means 48 and 50 which maintain shaft 42 slideable in a vertical upwards and downwards motion within chamber 36.

The handpiece assembly 11 of apparatus 10, when not in use, sits within sleeve 54 positioned on base 56 of the apparatus housing 12, as shown in figures 1 and 6. In this manner, potential damage to the handpiece 11 is minimised and it is retained in a convenient position for future use.

In order to use apparatus 10, cap 26 is removed to permit solutions, liquids or fluids to be poured into reservoir 22 to a desired level. The cap 26 is then securely replaced in circular port 24 to hermetically seal reservoir 22. The integral chamber 36 will retain air therein. In order to pressurised reservoir 22 and discharge the solution fluid or liquid through filtered discharge channel 32 in attachments means 38, the top piece 18 of the left portion 14 of housing 12 is manually pressed down over bottom piece 20. This motion forces plunger shaft 42 downward thereby forcing compressed air through the first check valve means 30 into reservoir 22. The air then rises above the surface of the solution, fluid or liquid in reservoir 22. Air pressure is thus created which acts to force the solution, fluid or liquid through filtered discharge channel 32 into tubing 40 to the handpiece assembly 11. The reservoir 22 will continue to discharge until the air pressure therein drops below the pressure necessary to discharge the solution, fluid or liquid to the handpiece assembly 11. In order to maintain the flow of the solution, fluid or liquid until the reservoir 22 is emptied, manual pressure should be continually provided by the operator of the apparatus 10 by continually pressing top piece 18 down over bottom piece 20 of housing 12.

The pressurised fluid dispensing apparatus of the present invention, when used properly as described above, provides a safe easy to use, supply of solution, fluid or liquid to a precision-controlled fluid placement device which may be utilised for a wide variety of medical, dental, commercial and industrial uses.

The foregoing is intended as illustrative of the present invention but not limiting. Numerous variations and modifications may be effected without departing from the true spirit and scope of the invention.

## Claims

1. A pressurised fluid dispensing apparatus for use with a precision-controlled fluid placement device comprising a reservoir fillable through a circular port, said circular port being closable by a removable cap, said reservoir being adapted to receive a plunger means and communicating with first check valve means for providing compressed air to dispense fluid from said reservoir through a filtered discharge channel, said channel being connected to second check valve means disposed in said fluid placement device to regulate flow of said fluid.

2. The pressurised fluid dispensing apparatus of claim 1 wherein said reservoir includes an integral chamber to receive said plunger means.

3. The pressurised fluid dispensing apparatus of claim 2 wherein said apparatus includes attachment means for securing said precision-controlled fluid placement device thereto, said attachment means being located at the lowest internal level of said chamber.

4. The pressurised fluid dispensing apparatus of claim 3 wherein said precision-controlled fluid placement device is a handpiece assembly.

5. The pressurised fluid dispensing apparatus of claim 4 wherein said attachment means conprises tubing.

6. The pressurised fluid dispensing apparatus of claim 4 wherein said attachment means conprises a nozzle.

7. The pressurised fluid dispensing apparatus of claim 2 wherein said plunger means includes a plunger shaft having a head, said shaft being slidable within said integral chamber.

8. The pressurised fluid dispensing apparatus of claim 7 wherein said plunger means is manually operated.

9. The pressurised fluid dispensing apparatus of claim 8 wherein said plunger shaft is disposed in said chamber between a plurality of generally cylindrical guide means for maintaining said shaft slidable within said chamber.

10. The pressurised fluid dispensing apparatus of claim 1 wherein said circular port is threaded to receive said removable cap, said cap being disposed to hermetically seal said reservoir.

11. In combination, a pressurised fluid dispensing means comprising at least one fluid reservoir chamber fillable through a port closable by a removable cap with bald reservoir chamber being adapted to employ a plunger means communicating with first check valve means for providing compressed air to discharge fluid from said reservoir chamber through a discharge channel to an attachment means to which a precision fluid placement device is connected.

12. The pressurised fluid dispensing means of claim 11 wherein said reservoir chamber includes an integral plunger chamber to receive said plunger means.

13. The pressurised fluid dispensing means of claim 11 wherein said fluid reservoir chamber includes a connector means to receive said plunger chamber with said plunger means disposed therein.

14. The pressurised fluid dispensing means of claim 11 wherein said discharge channel includes a particulate filtering means.

15. The pressurised fluid dispensing means of claim 12 wherein said apparatus includes attachment means for securing said precision fluid placement device thereto, said attachment means being preferably located near the lowest internal level of said reservoir chamber.

16. The pressurised fluid dispensing means of claim 15 wherein said attachment means includes an integral valve controlling fluid flow through said attachment means.

17. The pressurised fluid dispensing means of claim 12 wherein said plunger means includes a plunger shaft having head, said headed shaft being slidable with said integral chamber.

18. The pressurised fluid dispensing means of claim 17 wherein said plunger means is manually operated.

19. The pressurised fluid dispensing means of claim 17 wherein said plunger shaft is disposed in said chamber between a plurality of generally parallel guide means for maintaining said shaft slidably within said chamber.

20. The pressurised fluid dispensing means of claim 11 wherein said filling port in said reservoir chamber is circular and threaded to receive said removable rap, said cap being threaded and disposed to hermetically seal said reservoir chamber.

21. The pressurised fluid dispensing means of claim 20 wherein said threaded removable cap is provided with a filtered discharge tube of a length extending from said cap sealing said reservoir chamber to the lower internal level of said chamber.

22. The pressurised fluid dispensing means of claim 20 wherein said threaded removable cap has an attachment means for securing said precision fluid placement device to said cap.

23. The pressurised fluid dispensing means of claim 22 wherein said attachment means provides an integral valve controlling the flow of fluid through said attachment means.

24. The pressurised fluid dispensing means of claim 11 wherein said precision dispensing device is a handpiece assembly possessing a manually operated control valve.

25. The pressurised fluid dispensing means of claim 24 wherein said handpiece assembly comprises a handle and a distal nosepiece with an integral cannula disposed therein.

26. The pressure fluid dispensing means of claim 25 wherein said distal nose piece with an integral cannula is connected to said attachment means by a flexible tubing.

27. The pressurised fluid dispensing means of claim 24 wherein said handpiece assembly has a proximal connector means with a through bore continuous with the bore in said distal nose piece.

28. The pressurised fluid dispensing means of claim 27 wherein said distal nose piece has an attachment means to which removable cannulas or needles can be connected.

29. The pressurised fluid dispensing means of claim 28 wherein said proximal connector of said handpiece assembly employs a flexible tubing with proximal and distal connectors to connect said handpiece assembly securely to said attachment means of said pressurised fluid dispensing means.
